# EUROPEAN PATENT SPECIFICATION

(11) **EP 0 778 733 B1**
(45) Date of publication and mention of the grant of the patent: **20.12.2000**
(21) Application number: 95929788.8
(22) Date of filing: 01.09.1995
(51) Int. Cl.: A01N 63/00, C11D 3/386, C11D 3/48, C11D 3/38, A23L 3/3571, A23L 3/3526, A23L 3/3472

(54) **A BASIC PROTEIN COMPOSITION FOR KILLING OR INHIBITING MICROBIAL CELLS**
EINE GRUNDLEGENDE PROTEINZUSAMMENSETZUNG ZUR ABTÖTUNG ODER INHIBIERUNG MIKROBIELLER ZELLEN
COMPOSITION PROTEIQUE BASIQUE DESTINEE A TUER OU A INHIBER DES CELLULES MICROBIENNES

(30) Priority: 01.09.1994 DK 101194
(43) Date of publication of application: 18.06.1997
(73) Proprietor: NOVO NORDISK A/S, 2880 Bagsvaerd (DK)
(72) Inventor: JOHANSEN, Charlotte, DK-2880 Bagsvaerd (DK)
(86) International application number: DK9500351
(87) International publication number: WO9606532

(56) References cited:
- WO-A-90/03732
- WO-A-94/13774
- STN INTERNATIONAL, DERWENT INFORMATION LTD, WPIDS Accession No. 88-171767, ASAMA KASEI KK, "Food Preservative - Comprises Lysozyme, Gallic Acid Phyti Acid or Betaine and Eta-Poly Lysine"; & JP,A,63 109 762 (14-05-88) (8825).
- STN INTERNATIONAL, DERWENT INFORMATION LTD, WPIDS Accession No. 90-053418, QP CORP., "Alcohol Compsn. Used in Food Preservation and Disinfectant Materia - Contains Lower Fatty Acid Mono Glyceride, Protamine, Ethanol and Lysozyme or Acetic Acid"; & JP,A,02 002 329 (08-01-90) (9008).
- STN INTERNATIONAL, File JAPIO, JAPIO Accession No. 87-201563, NICHIRO GYOGYO KK., "Food Preservative"; & JP,A,62 201 563 (05-09-87), SHOWA.
- STN INTERNATIONAL, File JAPIO, JAPIO Accession No. 93-276910, NICHIRO CORP., "Food-Preserving Agent"; & JP,A,05 276 910 (26-10-93), HEISEI.
- STN INTERNATIONAL, DERWENT INFORMATION LTD, WPIDS Accession No. 80-85324C, LION FAT & OIL CO. LTD., "Detergent Compsn. Comprising Anionic and Nonionic Surfactants - Including Alpha Olefin Sulphonate and/or Polyoxyethylene Alkyl Ether Sulphuric Acid Ester"; & JP,A,55 133 495 (17-10-80) (8048).
- STN INTERNATIONAL, DERWENT INFORMATION LTD, WPIDS Accession No. 81-54186D, KIMURA Y., "Antimicrobial Compsn. Prepn. - by Adding Nonionic or Amphoteric Surfactants to Basic Peptide Antibiotic for Increased Activity"; & JP,A,56 068 616 (09-06-81) (8130).
- JOURNAL OF EXPERIMENTAL MEDICINE, Volume 108, 1958, JAMES G. et al., "Bactericidal Action of Histone", pages 925-944.
- PATENT ABSTRACTS OF JAPAN, Vol. 12, No. 70, C-479; & JP,A,62 209 005 (DASUKIN K.K.), 14 Sept. 1987.
- PATENT ABSTRACTS OF JAPAN, Vol. 13, No. 275, C-610; & JP,A,01 071 805 (RIKEN VITAMIN CO. LTD.), 16 March 1989.
- FOOD MICROBIOLOGY, Volume 11, No. 5, 1994, M. UYTTENDAELE et al., "Evaluation of the Antimicrobial Activity of Protamine", pages 417-427.
- MICROBIOLOGICAL REVIEWS, Volume 56, No. 3, Sept. 1992, MARTTI VAARA, "Agents That Increase the Permeability of the Outer Membrane", pages 395-411.

## Description

The present invention relates to a composition capable of killing microbial cells or inhibiting growing microbial cells, i.e. a bacteriocidal, bacteriostatic, fungicidal and/or fungistatic composition; a cleaning or detergent composition comprising a substance capable of killing microbial cells or inhibiting growing microbial cells; and methods for killing microbial cells present on a hard surface, on skin or in laundry, and for preserving food products, cosmetics etc.

### BACKGROUND OF THE INVENTION

At this time of increased public interest in reducing the use of chemical additives, it is relevant to consider natural alternatives for antimicrobial agents used e.g. for preserving foods, as desinfectants, and as an antimicrobial ingredient of detergent and cleaning compositions. This has increased interest in preservation using live bacterial cultures (Jeppesen & Huss 1993) and enzymes like lactoperoxidase (Farrag & Marth 1992), glucose oxidase (Jeong *et al*. 1992) and lysozyme (Johansen et al. 1994).

Protamines are basic proteins with a high arginine content found in association with DNA of spermatozoan nuclei of fish, birds, mammals etc. (Rodman *et al*. 1984; Kossel 1928). Protamine is used clinically as an antidote to heparin (Jaques 1973) and as a carrier of insulin, prolonging the absorption of subcutaneously administered insulin (Brange 1987). Attention has also been paid to the functional properties of protamine as a stabilizing agent (Phillips *et al*. 1989). Protamine has been shown to have an antibacterial effect (Hitsch 1958), but this aspect has not been thoroughly studied.

Synergistic combinations of protamines with polylysine or lysozyme are known from JP-A-63109762, JP-A-2002329 and JP-A-5276910.

The Gram-negative bacteria are often resistent to a large number of harmful agents due to the effective permeability barrier function of the outer membrane (Nakae 1985). However, protamine and most other cationic peptides are under certain conditions apparently able to traverse the outer membrane of Gram-negative bacteria (Vaara 1992, Vaara & Vaara 1983), probably as a result of their binding to the anionic lipopolysaccharide-covered surface of the Gram-negative cell. The mechanism of the antibacterial action of basic peptides is not known, but it has been suggested that they form a channel in the cytoplasmic membrane, thus uncoupling electron transport and causing leakage (Christensen *et al*. 1988; Hugo 1978; Kagan *et al*. 1990). It has also been proposed that they induce autolysis due to activation of the autolytic enzymes (Bierbaum & Sahl 1991).

In general, the occurance of highly-basic peptides such as protamine is relatively rare in nature. However, of those studied, several have been found to demonstrate antibacterial properties: eg. nisin (Sahl 1987), defensin (Lehrer *et al*. 1993), cecropins (Christensen *et al*. 1988), Pep5 (Bierbaum & Sahl 1987) and melittins (Vaara 1992).

Antibacterial activity is usually measured as a decrease in colony counts, a decrease in the absorbance of a bacterial suspension or as inhibition zones on agar plates (Trevors 1986). However, these methods may not be suitable when assaying the antibacterial effect of a basic peptide or protein such as protamine due to the agglutination of the positively-charged protamine and the negatively-charged bacterial cells as described by Islam *et al*. (1984).

Thus, the object of invention was to provide an antibacterial and/or antifungicidal composition comprising a natural active compound or substance, i.e. which is non-toxic, of biological origin, easily available and relatively inexpensive, optionally in combination with other antimicrobial compounds or substances.

### SUMMARY OF THE INVENTION

It has now surprisingly been found that it is possible to kill microbial cells or inhibit growing microbial cells by means of a basic protein or peptide of biological origin, e.g. protamine or protamine sulphate. For certain bacteria or fungi, it may be necessary to combine the basic protein with a cell-wall degrading enzyme or an oxidoreductase in order to obtain the desired antimicrobial effect.

Accordingly, the growth inhibitory effect of protamine and the potential lethal effect of this basic protein on non-growing cells has been investigated. Due to the methodological shortcomings described above, impedimetric measurements were used and compared to traditional plate counts (Firstenberg-Eden & Eden 1984; Connolly *et al*. 1993).

Thus, based these findings the present invention provides a bacteriocidal, bacteriostatic, fungicidal and/or fungistatic composition comprising a basic protein or peptide capable of killing microbial cells in combination with a cell-wall degrading enzyme or an oxidoreductase.

In another aspect, the present invention provides a detergent or cleaning composition comprising a basic protein or peptide capable of killing microbial cells and a surfactant. Such compositions have a pH in the alkaline range and it has been found that basic proteins such as protamine and protamine sulphate exhibit their optimum antimicrobial effect at alkaline pH, thus making such proteins very suitable for incorporation in compositions for cleaning purposes.

The composition of the invention is useful as antimicrobial ingredient wherever such an ingredient is needed, for example for the preservation of food, beverages, cosmetics, contact lens products, food ingredients or enzyme compositions; as a desinfectant for use e.g. on human or animal skin, mucous membranes, wounds, bruises or in the eye; for killing microbial cells in laundry; and for incorporation in cleaning compositions for hard surface cleaning.

### THE DRAWINGS

The invention is further illustrated by the drawings, in which
Figure 1 shows the effect of protamine on growth of *Yersinia enterocolitica* growing in TSB at 25°C. Growth is measured as % change in conductance;
Figure 2 shows calibration curves relating conductance ▲ (*Shewanella putrefaciens* strain A2) or capacitance ■ (*Listeria monocytogenes* strain O32) detection times in TSB at 25°C to colony counts in the absence of protamine;
Figure 3 shows the effect of various concentrations of protamine on the Gram-negative bacteria *Pseudomonas aeroginosa* in dependance of pH.
Figure 4 shows the effect of various concentrations of protamine on the Gram-positive bacteria *Listeria monocytogenes* in dependance of cell concentration (inoculum, log CFU/ml).
Figure 5 shows the effect of various concentrations of protamine on the Gram-negative bacteria *Shewanella putrefaciens* in dependance of cell concentration (inoculum, log CFU/ml).
Figure 6 is a response surface plot showing the synergistic effect of a composition of the invention (various concentrations of protamine and lysozyme) on the Gram-negative bacteria *Shewanella putrefaciens* in dependance of cell concentration (inoculum, log CFU/ml).

### DETAILED DESCRIPTION OF THE INVENTION

In the present context, the term "bacteriocidal" is to be understood as capable of killing bacterial cells.

In the present context, the term "bacteriostatic" is to be understood as capable of inhibiting bacterial growth, i.e. inhibiting growing bacterial cells.

In the present context, the term "fungicidal" is to be understood as capable of killing fungal cells.

In the present context, the term "fungistatic" is to be understood as capable of inhibiting fungal growth, i.e. inhibiting growing fungal cells.

The term "growing cell" is to be understood as a cell having access to a suitable nutrient and thus being capable of reproduction/propagation. By the term "non-growing cell" is meant a living, but dormant, cell, i.e. a cell in the non-growing, non-dividing, non-multiplying and non-energized state with metabolic processes at a minimum.

The term "cell-wall degrading enzyme" is to be understood as an enzyme which degrades components of the cell wall, e.g. peptidoglucans such as murein and pseudomurein; chitin; and teichoic acid. Examples of cell-wall degrading enzymes which are useful in compositions of the present invention are endoglycosidases Type II, e.g. the endoglycosidases Type II disclosed in EP-A2-0 425 018, lysozymes and chitinases.

The term "amino acids present in mammalian cells" denotes the 20 amino acids constituting the proteins being part of mammals, i.e. alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine. Preferably, the basic peptides or proteins of the composition of the invention consist of one or more of the mentioned 20 amino acids, i.e. the basic peptides or proteins may not be recovered from e.g. bacteria such as for example nisin and Pep5.

The term "oxidoreductase" means an enzyme classified as EC 1. according to the Enzyme Nomenclature (1992), i.e. any enzyme classified as EC 1.1 (acting on the CH-OH group of donors), EC 1.2 (acting on the aldehyde or oxo group of donors), EC 1.3 (acting on the CH-CH group of donors), EC 1.4 (acting on the CH-NH₂ group of donors), EC 1.5 (acting on the CH-NH group of donors), EC 1.6 (acting on NADH or NADPH), EC 1.7 (acting on other nitrogenous compounds as donors), EC 1.8 (acting on a sulfur group of donors) , EC 1.9 (acting on a heme group of donors), EC 1.10 (acting on diphenols and related substances as donors), EC 1.11 (acting on a peroxide as acceptor), EC 1.12 (acting on hydrogen as donor), EC 1.13 (acting on single donors with incorporation of molecular oxygen (oxygenases), EC 1.14 (acting on paired donors with incorporation of molecular oxygen), EC 1.15 (acting on superoxide radicals as acceptor), EC 1.16 (oxidizing matal ions), EC 1.17 (acting on -CH₂- groups), EC 1.18 (acting on reduced ferredoxin as donor), EC 1.19 (acting on reduced flavodoxin as donor), and EC 1.97 (other oxidoreductases).

The term "peroxidase enzyme system" is to be understood as a peroxidase (EC 1.11.1) in combination with a source of hydrogen peroxide which may be hydrogen peroxide or a hydrogen peroxide precursor for in situ production of hydrogen peroxide, e.g. percarbonate or perborate, or a hydrogen peroxide generating enzyme system, e.g. an oxidase and a substrate for the oxidase or an amino acid oxidase and a suitable amino acid, or a peroxycarboxylic acid or a salt thereof.

Examples of useful peroxidases are lactoperoxidase, horseradish peroxidase, peroxidases producible by cultivation of a peroxidase producing strain *Myxococcus virescens*, DSM 8593, *Myxococcus fulvus*, DSM 8969, or *Myxococcus xanthus*, DSM 8970, of a peroxidase producing strain of the genus *Corallococcus*, preferably belonging to *Corallococcus coralloides*, DSM 8967, or *Corallococcus exiguus*, DSM 8969.

In case of lactoperoxidase, thiocyanat may be used as a substrate.

Laccases are enzymes that catalyze the oxidation of a substrate with oxygen; they are known from microbial, plant and animal origins. More specifically, laccases (EC 1.10.3.2) are oxidoreductases that function with molecular oxygen as electron acceptor. Molecular oxygen from the atmosphere will usually be present in sufficient quantity, so normally it is not necessary to add extra oxygen to the process medium. Examples of a laccase enzyme useful in the compositions of the present invention is laccase obtainable from the strain *Coprinus cinereus*, IFO30116, or from a laccase having immunochemical properties identical to those of a laccase derived front *Coprinus cinereus*, IFO30116; or obtainable from a strain of *Myceliophthora thermophile* as disclosed in WO 91/05839.

The term "microbial cells" denotes bacterial or fungal cells.

By the term "of biological origin" is to be understood that the substance or compounds is recovered or regenerated from biological material such as humans, animals or plants. Similarly, the term "of microbiological origin" denotes that the substance or compounds is recovered or regenerated from microbiological material such as bacteria, fungi, yeast or that a parent or native substance or compound is producible by a microbiological organism.

The term "biological material" denotes living material obtainable from Nature or previously living material obtainable from Nature.

The term "synthesized polypeptide" denotes a synthesized assembly, i.e. a chain, built of peptide monomers. Polypeptides which are useful in the present compositions are basic polypeptides, i.e. polylysins and polyarginins and co-polymers thereof. It is preferred that the polypeptides have a chain length of less than about 100 amino acids but it is contemplated that polypeptides of less than about 1000 kD are useful. Preferably, the polypeptides to be used in the composition of the invention is of almost identical chain length or molecular weight but mixtures of polypeptides having various chain lengths or molecular weights are also useful.

It is contemplated that the basic protein of the composition of the invention may be a recombinant protein. In case of protamine, it is contemplated that the protamine may be a recombinant protamine, i.e. produced by cloning of a DNA sequence encoding the protein and subsequent cell transformed with the DNA sequence and expressed in a host, i.e. a suitable fungal or bacterial host. A recombinant protamine/protamine sulphate may be cloned and expressed according to standard techniques conventional to the skilled person.

Preferred basic proteins to be used in the compositions of the present invention are protamines, protamine sulphates, defensins, magainins, melittin, cecropins and protegrins; more preferably protamines and protamine sulphates.

Hitherto it has been known that protamine from salmon has a bacteriocidal effect on growing Gram-positive bacteria (1000 µg/ml). Islam *et al*. (1984) found that it inhibited growth but did not determine whether the effect was bacteriocidal or bacteriostatic. Other studies have reported that protamine is not effective against Gram-negative bacteria (Islam *et al*. 1984; Yanagimoto *et al*. 1992). Contrary to this observation, it has now been found that protamine is effective against Gram-positive bacteria, Gram-negative bacteria and fungi. The same applies for protamine sulphate.

It has been suggested the primary antibacterial effect of many of the basic peptides is their ability to penetrate the cytoplasmatic membrane, disrupting the electron transport and induce leakage of intracellular compounds. Without being bound to this theory, this mechanism may explain in part the effect of protamine on some of the strains tested in the experiments described in the Examples below.

In another aspect, the present invention relates to a cleaning or detergent composition comprising a basic protein or peptide capable of killing microbial cells and a surfactant.

The detergent or cleaning composition may further comprise other enzymes conventionally used in detergent or cleaning compositions. Preferably, the detergent or cleaning composition of the invention comprises at least one enzyme selected from the group consisting of proteases, amylases, cellulases, and lipases.

The surfactant of the detergent or cleaning composition is preferably a detergent surfactant, more preferably a detergent surfactant selected from the group consisting of anionic, nonionic, ampholytic, zwitterionic and cationic surfactants.

In a preferred emboodiment, the detergent or cleaning composition comprises as the basic protein a protamine or a protamine sulphate in an amount effective for killing cells or inhibiting growth of cells, preferably in an amount corresponding to between 1 and 4000 µg per ml cleaning liquor or washing liquor, more preferably between 1 and 2000 µg per ml cleaning liquor or washing liquor, especially between 5 and 1000 µg per ml cleaning liquor or washing liquor.

In a further aspect, the present invention relates to the use of the compositions of the invention for various purposes, i.e. the invention also relates to a method for killing microbial cells present on a hard surface which method comprises contacting the surface with a cleaning composition of the invention, preferably a composition comprising a protamine or a protamine sulfate, in an amount effective for killing the cells.

Also, in yet another aspect the invention relates to a method for killing microbial cells or inhibiting growing microbial cells present on laundry which method comprises contacting the laundry with a detergent composition of the present invention, preferably a composition comprising a protamine or a protamine sulfate, in an amount effective for killing the cells or for inhibiting growing cells.

In yet another aspect the invention relates to a method for preservation of food, beverages, cosmetics such as lotions, creams, gels, soaps, shampoos, conditioners, antiperspirants; contact lens products, food ingredients or enzyme compositions which method comprises incorporating into the unpreserved food, beverages, cosmetics, contact lens products, food ingredients or enzyme compositions a basic protein or basic peptide or a composition of the present invention in an amount effective for inhibiting growing microbial cells, preferably a protamine or a protamine sulphate or a composition comprising a protamine or a protamine sulphate.

In yet another aspect the invention relates to a method of killing microbial cells present on human or animal skin, mucous membranes, wounds, bruises or in the eye which method comprises contacting the cells to be killed with a basic protein or peptide in an amount effective for killing the cells, preferably a protamine or protamine sulphate. Thus, the compositions of the invention and/or the basic peptides or proteins used in these compositions, especially protamines and protamine sulphates, may by useful as desinfectants, e.g in the treatment of acne, infections in the eye, skin infections; in antiperspirants; for cleaning end desinfection of contact lenses etc.

The invention is further illustrated by the following non-limiting examples.

### EXAMPLE 1

### Bacteriocidal and bacteriostatic effect of protamine

### Materials and Methods

### BACTERIA, INOCULUM, MEDIA AND REAGENTS

The bacteria used in the study are listed in Table 1 below. Stock cultures were maintained in Tryptone Soy Broth (TSB) (Oxoid CM129) with 0.5% glucose, 2% skimmed milk powder, 4% glycerol and stored at -80°C.

Cells from the stock culture were streaked on TSB plates (TSB with 1.2% agar) and incubated 48 h at 25°C. One colony of bacteria was inoculated in 5 ml of TSB and grown 24-36 h at 25°C. This culture was used as inoculum.

TSB with 1.2% agar was used for plate counts which were done by surface inoculation and incubation of the plates at 25°C. Ten-fold dilution rates were prepared using sterile peptone saline (0.1% peptone, 0.85% NaCl).

Protamine from Salmon (P-4005) was obtained from Sigma Chemical Company (St. Louis, USA), dissolved in distilled water, filter sterilized (0.2 µm) and used immediately after preparation.

**Table 1**

| Bacterial strains tested for sensitivity to protamine | | |
|---|---|---|
| Bacteria (reference) | Gram reaction | Code (ref.) |
| *Aeromonas sobria* (g) | - | F4 |
| *Aeromonas salmonicidae* (a) | - | AS1 |
| *Pseudomonas fluorescens* (h) | - | AH2 |
| *Shewanella putrefaciens* | - | A2/A6/- |
| (j)/(k)/(k)/(k) | | A11/A22 |
| *Vibrio anguillarum* (b) | - | E2 |
| *Vibrio paraheamolyticus* (b) | - | VP |
| *Yersinia enterocolitica* (c) | - | I1 |
| *Brochotrix thermosphacta* (d) | + | BT |
| *Listeria monocytogenes* (e)/(m) | + | O32/O57 |
| *Staphylococcus aureus* (d)/(f) | + | M2/SA |
| *Escherichia coli* (b) | - | EC |
| *Pseudomonas aeruginosa* (f) | - | PA |
| *Bacillus subtilis* (n) | + | ATCC 6633 |
| *Corynebacterium jeikeium* (f) | + | CJ |
| Strains obtained from: (a) Fish Pathology Laboratory, Royal Veterinary and Agricultural University, Denmark. (b) Dept. for Veterinary Microbiology and Hygiene, Royal Veterinary and Agricultural University, Denmark. (c) Environmental and Food Laboratory, Skovlunde, Denmark. (d) Department of Biotechnology, Technical University of Denmark. (e) Campden Food and Drink Association, Chipping Campden, United Kingdom. (f) Department of Clinical Microbiology, Statens Seruminstitut, Denmark (g) Knøchel, 1989. (h) Gram *et al.*, 1990. (j) Jørgensen, 1986. (k) Jørgensen and Huss, 1989. (m) Ben Embarek and Huss 1993. (n) Chung, Steen and Hansen, 1994. | | |

### IMPEDANCE DETERMINATIONS

Volumes (1.0 ml) of TSB medium were added to Bactometer® wells. Protamine solutions (0.1 ml) were transferred to the wells which were sealed, connected to the Bactometer B123-2 (bioMérieux UK ltd., UK) and incubated at 25°C until a significant detectable increase in the electrical conductivity of the medium was registered and the detection time (DT) was recorded (maximum 100 h). Detection usually occurs when the cell concentration reaches 10⁶-10⁷ cfu/ml. For the Gram-positive strains the capacitance signal was monitored, while change in conductance was used for Gram-negative strains.

### EFFECT OF PROTAMINE ON GROWING CELLS The antibacterial activity of protamine on growing cells

was measured in the Bactometer modules. The wells (containing 1 ml TSB and 0.1 ml protamine solution) were inoculated with 0.1 ml from a 10⁻⁴ dilution of an inoculum culture, giving a final cell concentration in the well of approximately 10³ cfu/ml. The concentration of protamine varied from 1 to 4000 µg/ml depending on the sensitivity of the strain investigated.

Minimum Inhibition Concentration (MIC) was determined as the lowest concentration of protamine resulting in absence of a DT.

When no DT was measured, the lethal/inhibitory effect on the cells were tested by plating the total well volume.

### EFFECT OF PROTAMINE ON NON-GROWING CELLS

1 ml of inoculum diluted to 10⁻³ was inoculated in 250 ml TSB (approximately 10³ cfu/ml) and incubated at 25°C for 24 h. Cells were harvested by centrifugation (2000*g for 10 min), washed twice with 0.067 M sterile sodium phosphate buffer pH 7.0 (Weisner 1984) and resuspended in the same buffer. The absorbance at 450 nm (OD₄₅₀) of the bacterial suspension was adjusted to 1.0 (approximately 10⁸ cfu/ml), measured on a Perkin Elmer Lambda 2 spectrophotometer (Überlingen, Germany). The cell suspension was diluted in sterile phosphate buffer to concentrations of 10⁵ and 10³ cfu/ml. Protamine was added to the cell suspensions (10³, 10⁵ and 10⁸ cfu/ml) in concentrations of 0, 50, 100 and 500 µg/ml, and the suspensions were incubated at 25°C for 24 h.

### CALIBRATION CURVES

A series 10-fold dilution rate was prepared from the 10⁸ cfu/ml suspension with no protamine added. A calibration curve relating cfu/ml of the 10-fold dilutions to capacitance or conductance DT in TSB was constructed for each strain using a minimum of 8 dilution steps.

From the protamine treated suspensions, 0.1 ml was inoculated in TSB in Bactometer wells and the DT determined. This DT was, converted to a colony count using the calibration curve. Thus, colony counts were not made directly on the protamine treated suspensions as protamine caused significant clumping of the bacterial cells.

When no DT was measured, the total well volume was pipetted onto agar plates to evaluate whether protamine had a bacteriostatic or bacteriocidal effect.

### Results

The MIC values determined from capacitance or conductance DT of cells growing in TSB are shown in Table 2.

**Table 2**

| **Minimum inhibition concentration (MIC) measured impedimetric as a total inhibition after 100 hours at 25°C.** | |
|---|---|
| Strain | MIC (µg/ml) |
| *Aeromonas sobria* | > 4000 |
| *Aeromonas salmonicidae* | 4000 |
| *Escherichia coli 0157:H7* | 1000 |
| *Pseudomonas aeruginosa* | 4000 |
| *Pseudomonas fluorescens* | 3000 |
| *Shewanella putrefaciens* (4 strains) | 500-1000 |
| *Vibrio anguillarum* | 1000 |
| *Vibrio paraheamolyticus* | 500-1000 |
| *Yersinia enterocolitica* | >4000 |
| *Bacillus subtilis* | 100 |
| *Brochotrix thermosphacta* | 20 |
| Corynebacterium jeikeium | 100 |
| *Listeria monocytogenes* (2 strains) | 1000 |
| *Staphylococcus aureus* (2 strains) | 500-1000 |

The Gram-positive strains were more sensitive to protamine than the Gram-negative. The MIC values determined for Gram-positive strains varied from 20 to 1000 µg/ml and varied from 500 µg/ml to more than 4000 µg/ml for the Gram-negative strains.

*Brochotrix thermosphacta* was the most sensitive strain, and a protamine concentration of 20 µg/ml TSB caused a total kill of the inoculum (10³ cfu/ml), measured by plating the well volume after 100 h of incubation in the Bactometer. A protamine concentration of 1000 µg/ml resulted in a 100% lethal effect on the two strains of *Listeria monocytogenes* and *Staphylococcus aureus* (10³ cfu/ml). The MIC for protamine on *S. aureus* was 500 µg/ml, however, this concentration did not have a lethal effect.

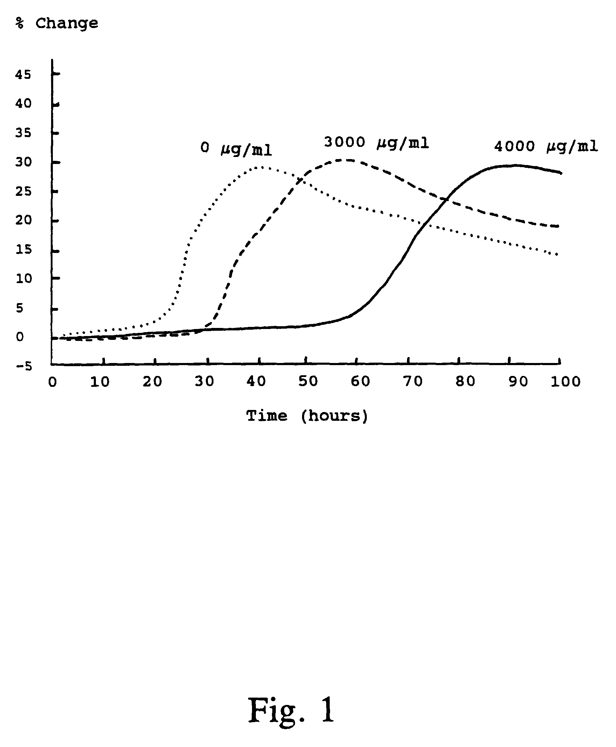

The DT's for *Aeromonas sobria* and *Yersinia enterocolitica* increased with increasing protamine concentration, suggesting a prolonged lagphase. However, the cultures were not totally inhibited and no MIC was determined. The DT for a 10³ cfu/ml suspension of *A. sobria* was 33 h when incubated with 4000 µg/ml compared to 12 h when no protamine was added. For *Y. enterocolitica* the detection time of 10³ cfu/ml was prolonged from 21 h to 60 h when 4000 µg/ml of protamine was added, see Figure 1. *Aeromonas salmonicidae* and *Pseudomonas fluorescens* were inhibited by protamine in concentrations of 4000 and 3000 µg/ml TSB, respectively. Thus, no change in conductance was seen after 100 hours incubation, but live cells were isolated from the well. *Shewanella putrefaciens* (A2), (A11) and (A22) and *Vibrio anguillarum* were inhibited by protamine in the concentration 1000 µg/ml TSB, and *Vibrio paraheamolyticus* and *S. putrefaciens* (A6) were inhibited by 500 µg/ml TSB. *S. putrefaciens* was the only Gram-negative bacteria which was killed by protamine, thus, 2000 µg/ml of protamine killed 100% of the inoculum (10³ cfu/ml) of all the four tested strains.

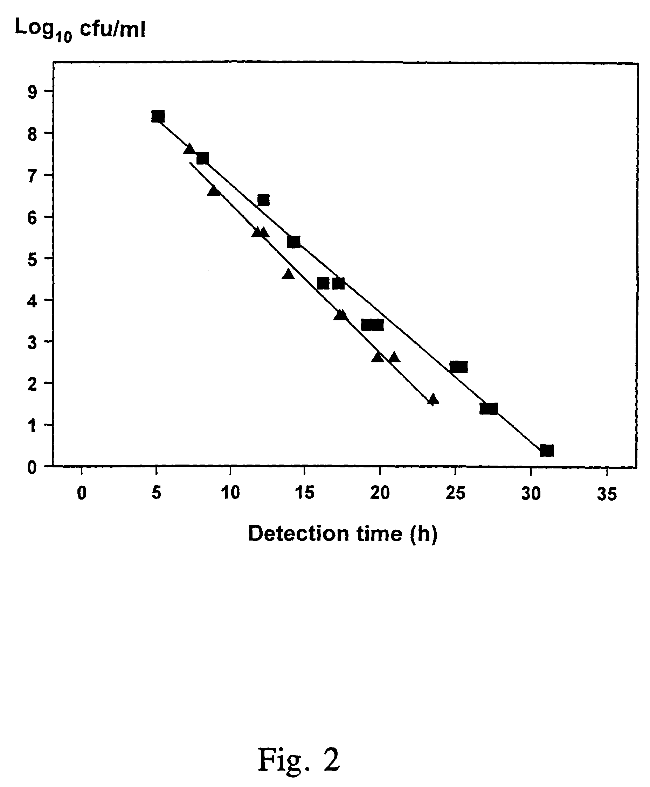

The bacteriocidal effect of protamine on non-growing cells was tested on four strains of *S. putrefaciens* and two strains of *L. monocytogenes*. After protamine treatment, detection times were measured and converted to a cell count using the calibration curve generated for the particulary strain (Fig. 2). The sensitivity of *S. putrefaciens* varied from strain to strain, see Table 3 below (see also table 1 for code and reference for each strain). Strain A2 and A11 were similar in sensitivity and more resistant than A6 and A22. Thus, 100 µg protamine/ml killed *S. putrefaciens* strain A6 and A22 when suspended at low cell concentrations (10⁵ and 10³ cfu/ml). The same level was not 100% lethal on *S*. *putrefaciens* strain A2 and A11, however the cell number was reduced by 90-99.9%.

The initial cell number was estimated by absorcance measurements.

**Table 3**

| Number of bacterial cells surviving 24 h protamine treatment as estimated from capacitance/conductance calibration curves. | | | | | |
|---|---|---|---|---|---|
| Bacteria | Code | Initial cell number (cfu/ml) | Estimated cell number after 24 h of protamine treatment in 4 concentrations of protamine (µg/ml) | | |
| | | | 0 | 100 | 500 |
| *S*. *putrefaciens* | A2 | 10⁸ | 6*10⁸ | 6*10⁵ | 8*10⁶ |
| | | 10⁵ | 8*10⁶ | ∼1^{c} | ∼1^{c} |
| | | 10³ | 1*10⁴ | ∼1^{c} | ∼1^{c} |
| - | A6 | 10⁸ | 1*10⁸ | 8*10³ | 4*10¹ |
| | | 10⁵ | 8*10⁴ | k | k |
| | | 10³ | 3*10³ | k | k |
| - | A11 | 10⁸ | 5*10⁸ | 3*10⁶ | 2*10² |
| | | 10⁵ | 2*10⁶ | 3 | 1 |
| | | 10³ | 3*10² | ∼1^{c} | ∼1^{c} |
| - | A22 | 10⁸ | 6*10⁸ | 2*10⁷ | 1*10⁵ |
| | | 10⁵ | 6*10⁴ | k | k |
| | | 10³ | 3*10³ | k | k |
| *Listeria monocytogenes* | O32 | 10⁸ | 9*10⁸ | 8*10⁸ | 1*10⁶ |
| | | 10⁵ | 8*10⁶ | 1*10⁶ | 6*10⁵ |
| | | 10³ | 2*10⁴ | 4*10⁴ | 5*10⁴ |
| - | O57 | 10⁸ | 3*10⁸ | 4*10⁸ | 3*10⁸ |
| | | 10⁵ | 1*10⁶ | 8*10⁴ | 3*10⁵ |
| | | 10³ | 5*10⁴ | 2*10² | 3*10² |
| k: No surviving cells (determined by spread plating the medium from the well. c): Long detection time (DT) corresponding to a very low cell number (approximately 1 surviving cell). | | | | | |

Protamine at 100 and 500 µg/ml had no effect on non-growing *L. monocytogenes* cells, and increasing the protamine concentration to 1000 µg/ml did not cause any lethal effect of protamine on non-growing *L. monocytogenes*.

The results show that salmine (salmon protamine) in concentrations of 100-4000 µg/ml prolonged the lag phase of several Gram-negative bacteria significantly. Protamine was more effective on actively growing *L. monocytogenes* cells as compared to cells suspended in buffer. The bactericidal effect of protamine on *Shewanella putrefaciens* was seen on both growing and non-growing cells. A protamine concentration of 2000 µg/ml was required to kill growing *S. putrefaciens* (10³ cfu/ml), whereas non-growing cells were killed by only 50 µg/ml. When the cell concentration was raised the bactericidal effect of protamine was decreased, probably as a result of the higher cell/protamine ratio.

The impedimetric method used in this study proved useful for the measurement of the antibacterial activity of a cationic protein which caused cellular agglutination. Excellent correlations exit between detection time and cfu of untreated cells, see Figure 2. The correlation between protamine treated cells and cfu was statistically similar to the correlation for untreated cells (data not shown), however, plating the protamine treated cells caused a great degree of variation on the cells count. It is demonstrated that protamine inhibits growth of all the tested strains, determined as a prolonged lag phase for the most resistant bacteria or a lethal effect on a few of the tested strains, the Gram-positive bacteria in particular. The fact that protamine is naturally occurring and non-toxic makes it an antibacterial protein that might hold great promise for the control of e.g. spoilage bacteria and food-borne pathogenes.

### EXAMPLE 2

### Comparison af minimum inhibition concentrations for basic proteins and enzymes

The minimum inhibition concentration (MIC) of various-substances was determined as described in Example 1.

The following substances were tested: protamine (A), protamine sulphate (B), a peroxidase enzyme system (i.e. lactoperoxidase/glucoseoxidase (C)), subtilisin A (D), polyarginine (E) having an average molecular weight of about 6 kD and lysozyme (F) (150 000 units/mg; Johansen, C. et al., 1994). The results are shown in Table 4 below.

It is demonstrated that protamine and protamine sulphate are very effective substances for inhibiting all the tested strains, whereas polyarginine is effective for inhibiting all strains but *Pseudomonas spp.*. Apart from the effect of lysozyme on *Listeria monocytogenes*, none of the tested enzyme showed any effect.

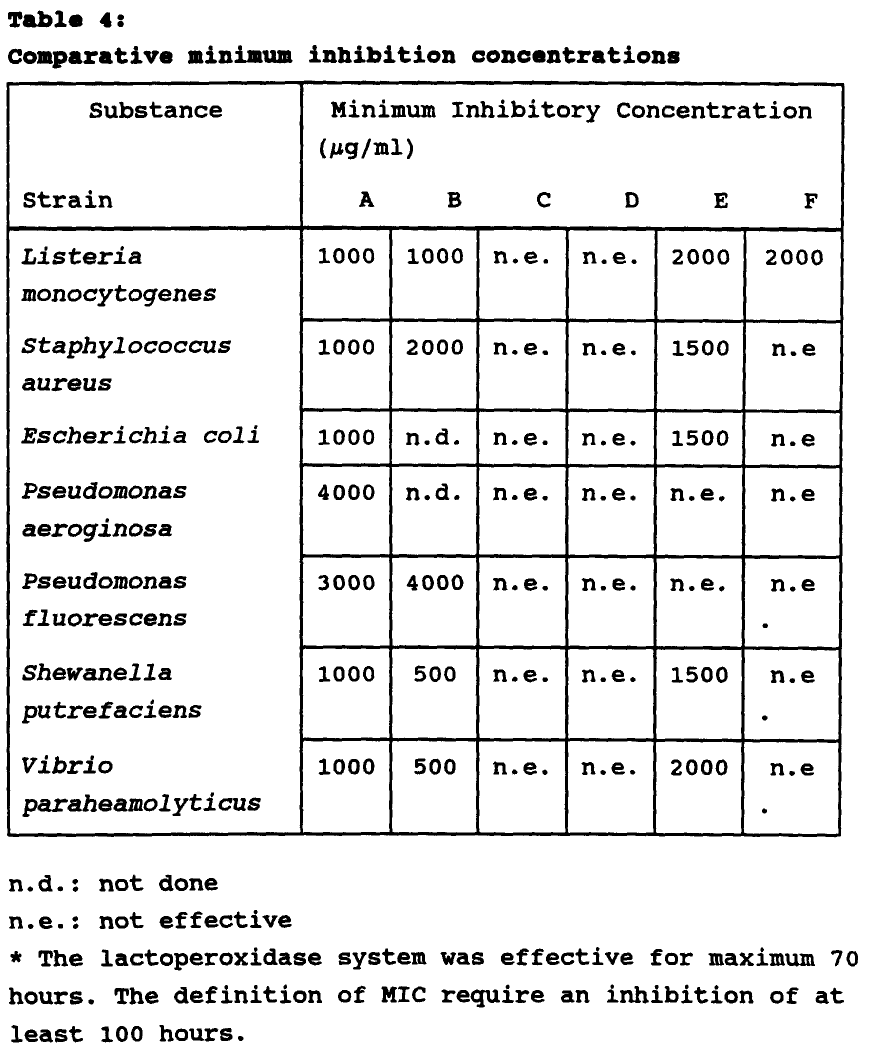

### EXAMPLE 3

### The influence of pH and cell concentration on the antibacterial effect of protamine

The influence of pH on the antibacterial effect of protamine was tested using the materials and methods described in Example 1.

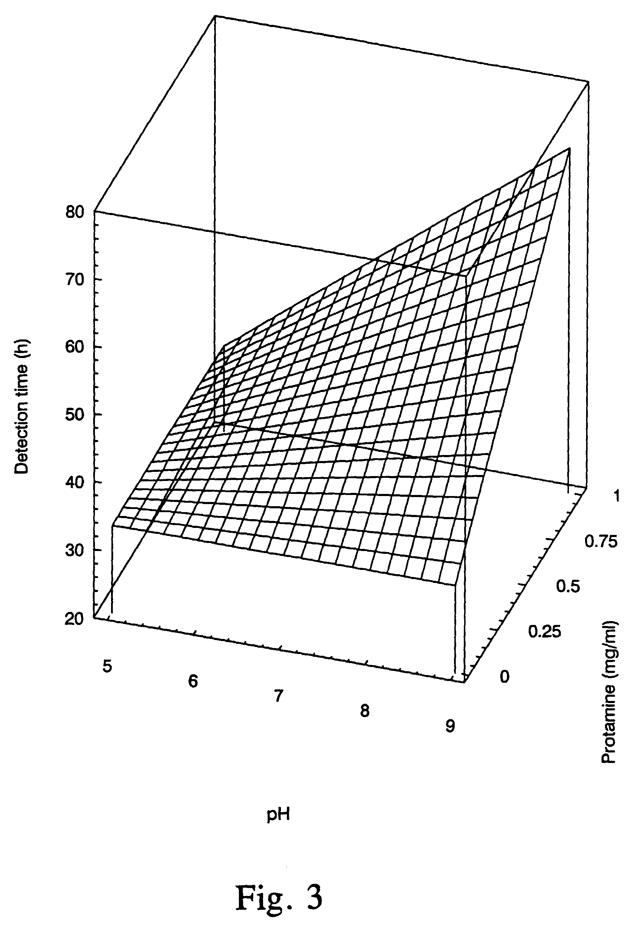

The results are shown in Figure 3 and demonstrate clearly that the antibacterial effect of protamine depend significantly on pH. At low pH, protamine (1 mg/ml) has no effect on the Gram-negative bacteria *Pseudomonas aerouginosa*, however, at high pH protamine (1 mg/ml) prolonged the detection time from 32 to 71 hours. The interaction between pH and protamine has been observed for all the tested strains.

Further, the influence of cell concentration on the anti-bacterial effect of protamine was tested using the materials and methods described in Example 1, i.e.the correlation between cell concentration and protamine concentration has been measured by the impedimetric assay.

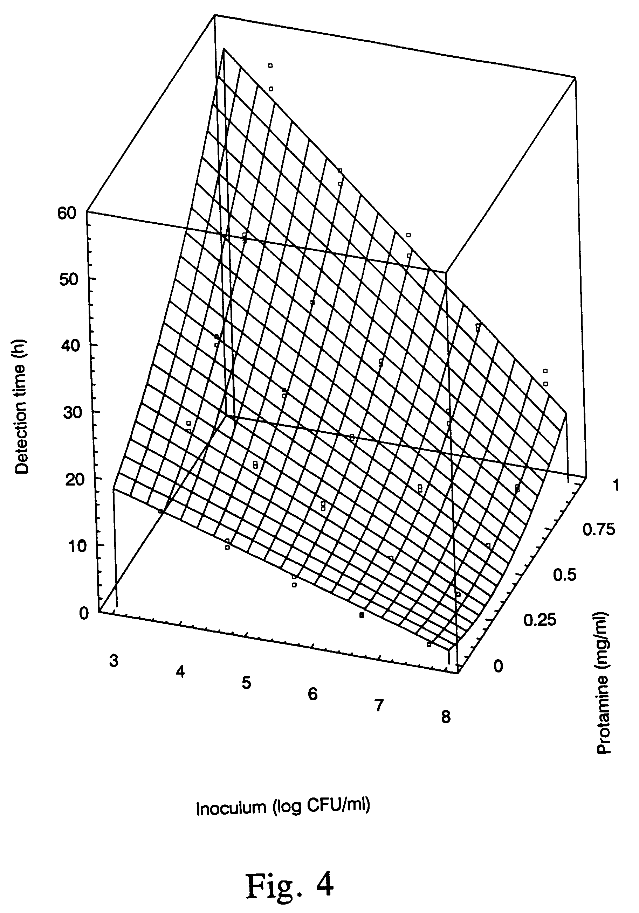

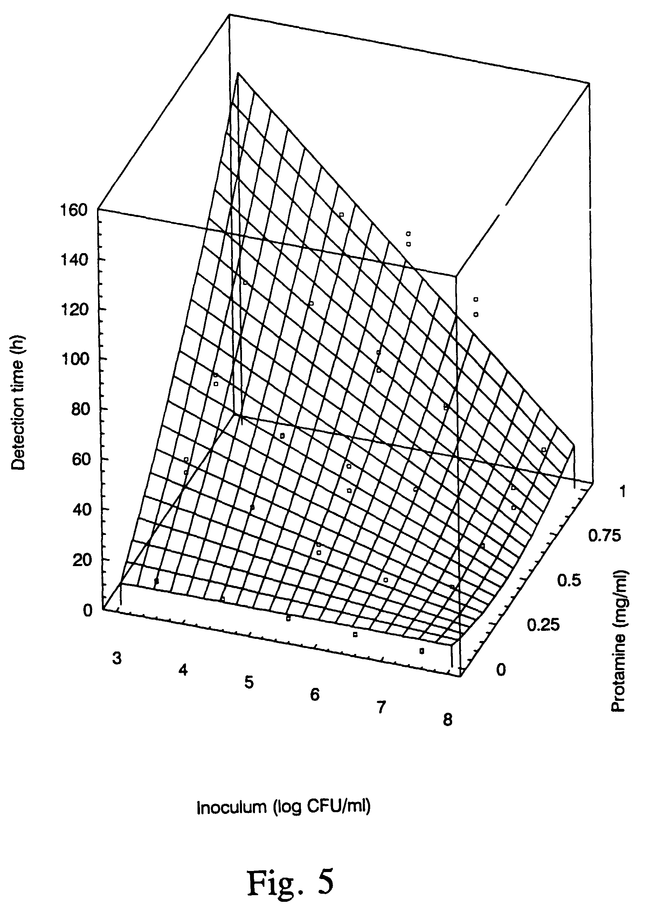

The results are shown in Figure 4 and Figure 5 and demonstrate clearly that a significant synergistic effect between the cell concentration and the protamine concentration has been observed. Thus, at low cell concentration, protamine (1 mg/ml) caused a prolongation of the detection time from 12 to above 100 hours for the Gram-negative bacteria *Shewanella putrefaciens* (4 strains), compared to a prolongation at high cell concentration from 6 to 18 hours. The detection time for the Gram-positive bacteria *Listeria monocytogenes* (2 strains) was at low cell concentration prolonged from 18 to 55 hours when treated with protamine (1 mg/ml), at high cell concentration the same protamine concentration only prolonged the detection time from 4 to 15 hours.

### EXAMPLE 4

### Synergistic antimicrobial effect between a basic protein, a cell-wall degrading enzyme and a peroxidase enzyme system

Impedimetric measurements carried out as described in example 1 have shown an synergistic effect between basic peptides as protamine, polyarginine or polylysine and lysozyme and/or glucose oxidase and/or the lactoperoxidase enzyme system depending on pH and NaCl concentration.

Growth inhibition experiments were conducted, wherein synergistic and additional effects were determined by mixing compounds in low concentrations not having any activity on their own and using a factoral design. The effects were measured as growth inhibition or a 100% bacteriocidal effect with a total kill of the inoculum.

Protamine (250 µg/ml) or polylysine (500 µg/ml) in combination with lactoperoxidase (2 U/ml) and glucose oxidase (2 U/ml) had a 100% lethal effect on *Pseudomonas fluorescens*, whereas the same strain was not inhibited when treated with any of these three compounds alone in the concentrations mentioned above.

A synergistic effect was as observed against *Pseudomonas fluorescens* when combining protamine (250 µg/ml) and polylysine (500 µg/ml) and lysozyme (50000 U/ml) and lactoperoxidase (2 U/ml) and glucose oxidase (1 U/ml) or lysozyme (50.000 U/ml) and polylysine (500 µg/ml) and lactoperoxidase (2 U/ml) and glucose oxidase (1 U/ml).

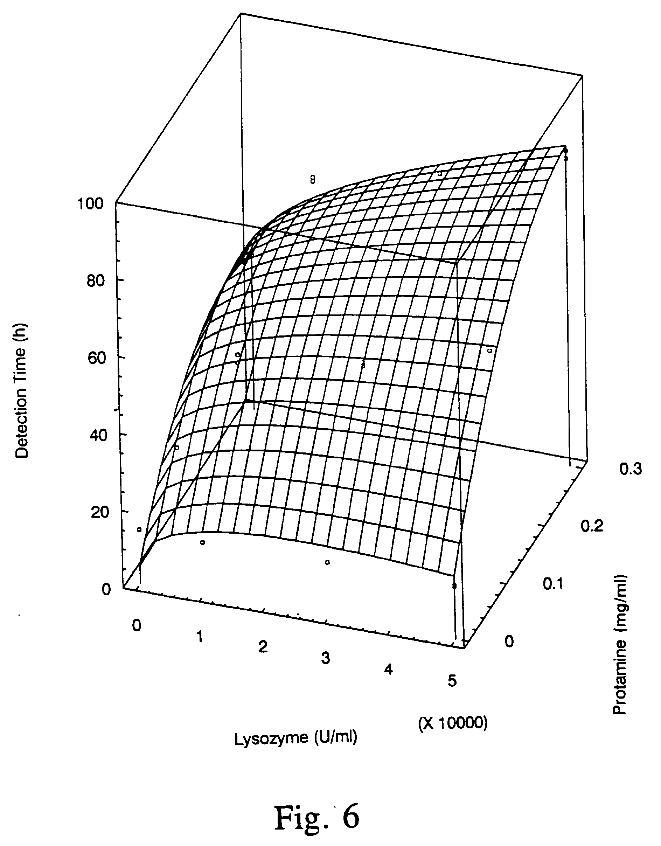

Experiments where the antibacterial effect was measured as growth inhibition of *Shewanella putrefaciens* in TSB at 25°C and pH 7.2, showed a synergistic effect between protamine and lysozyme; the results are shown in Figure 6 as a response surface plot. Lysozyme alone had no effect on the Gram-negative bacteria *Shewanella putrefaciens* neither had protamine at concentrations below 500 µg/ml, whereas combinations caused a 100% bactericidal activity at protamine concentrations above 300 µg/ml and lysozyme concentrations from 10⁴-10⁶ U/ml (lysozyme activity: 150000 U/mg).

### EXAMPLE 5

### Fungistatic and fungicidal activities

This experiment was carried out as described in example 1 using the Bactometer substrate: 0.75 g Yeast extract (Difco), 3.0 g D(+)Glucose, 1 g KH₂PO₄, 0.8 g isogel agarose IEF (Pharmacia) and 100 ml distilled water.

Protamine was added to the substrate immediately before inoculation with a spore suspension of the test fungi (approximately 10³-10⁴ cfu/ml).

The minimum inhibitory concentration was determined as the lowest concentration of protamine resulting in a absence of DT during 100 h measurement (see table 5 below). When no DT was determined a fungicidal activity was evaluated by plating from a dilution of the total well volume.

**Table 5**

| Strain | pH | MIC (µg/ml) |
|---|---|---|
| *Alternaria infectoria* | 5.2 | 240 |
| *Aspergillus niger* | 7.1 | 1000 |
| *Botrytis aclada* | 5.2 | 120 |
| *Cladosporium herbarum* | 5.2 | 120 |
| *Eurotium repens* | 5.2 | 240 |
| *Fusarium culmorum* | 5.2 | 240 |
| *Penicillium comcam* | 6.1 | 1000 |
| *Penicillium crustosum* | 6.2 | 1000 |
| *Penicillium roqueforti* | 5.2 | 240 |
| *Ulocladium atrum* | 5.2 | 240 |

The fungistatic and fungicidal effect of protamine was optimal at high pH and low inoculum size as the effect on bacteria. Increasing the pH caused a significant decrease in the MIC-value. A fungistatic effect was obtained with an 2-5 fold lower protamine concentration than used when a fungicidal effect was determined. The most resistant strains shown in table 5 were not inhibited by protamine for 100 h at low pH, thus a 100 h inhibition was not obtained before increasing the pH to the values given in the table.

### EXAMPLE 6

### Survival and transfer of bacteria during mini-wash

### Materials:

Ariel Color (DF-9412330).
Swatches (white cotton, DF-9415585), sterilized by Tryptone Soya Broth (TSB).
Sterilized water (12°dH).

### Strains:

*Staphylococcus aureus* (skin isolate)
*Pseudomonas aeruginosa* (skin isolate)

### Methods:

### Inoculum:

*S. aureus* and *P. aeruginosa* were grown in Tryptone Soya Broth (TSB) at 25°C for 30 hours. For each strain six sterile swatches were inoculated with approximately 10⁶ cfu/swatch and air dried for 30 minutes.

### Mini-wash:

0.56 g of Ariel Color was dissolved in 80 ml sterile water (12°dH, 35°C) in each wash beaker, giving the final concentration of 7 g/l. Protamine, dissolved in water and filter sterilized, were added to half of the wash beakers giving the final concentration of 500 µg/ml.

After 70 sec. 1 inoculated swatch and 2 sterile swatches were transferred to each wash beaker, and washed at 35°C for 15 min. In one beaker 3 sterile swatches were washed as control.

From each wash beaker 0.1 ml of detergent solution were transferred to Malthus (in-direct cells) containing TSB and incubated.

The swatches were rinsed in sterile water for 10 min (stirring). From each beaker 0.1 ml of rinse water were incubated in Malthus (in-direct cells). were incubated in Malthus (in-direct cells).

After wash all swatches were slightly air dried in sterile air for 10 min, and each swatch was transferred to an in-direct Malthus cell.

All materials and instruments except the detergent, were sterilized before use to avoid contamination.

### In-direct Malthus:

Indirect-Malthus measurements were used when estimating the number of viable cells.

3 ml of TSB were transferred to the outer chamber of the in-direct Malthus cells, and 0.5 ml of sterile KOH (0.1 M) were transferred to the inner chamber. As cells are growing in the outer chamber they produce CO₂ (g) which will dissolve in the KOH in the inner chamber and thereby change the conductance of the KOH. When the conductance change is measurable by the Malthus, a detection time (DT) will be recorded. The DT's were converted to colony counts by use of a calibration curve relating cfu/ml to DT (see Figure 1 and 2).

A series 10-fold dilution rate was prepared from the 10⁸ cfu/ml suspension of cells. Conductance DT of each dilution step was determined in TSB, and a calibration curve relating cfu/ml of the 10 fold dilutions to DT in TSB was constructed for each strain (see Figure 1 and 2).

### Results:

The number of cells surviving mini-wash, in the detergent solution, attached to the contaminated swatches, transferred to the rinse water or to the sterile swatches, were determined by in-direct Malthus (table 6).

A relatively high number of cells were washed of the swatches and found in the wash water, however, approximately 10³ cfu were still attached to the swatches after mini-wash and rinsing for 10 min, and during wash cells were transferred from the contaminated swatch to the sterile swatches. The *S. aureus* cells were found very sensitive to protamine, which can be explained by the increased antibacterial activity of protamine at high pH. A high number of *P. aeruginosa* were determined in the detergent solution, and protamine was found active against the cells in the detergent solution, however, *P*. *aeruginosa* attached to the swatches were not inhibited or killed by protamine, and all the sterile swatches in the washes inoculated with *P. aeruginosa* were contaminated during wash.

The rinse water were almost sterile, thus the cells may adhere actively to the textile.

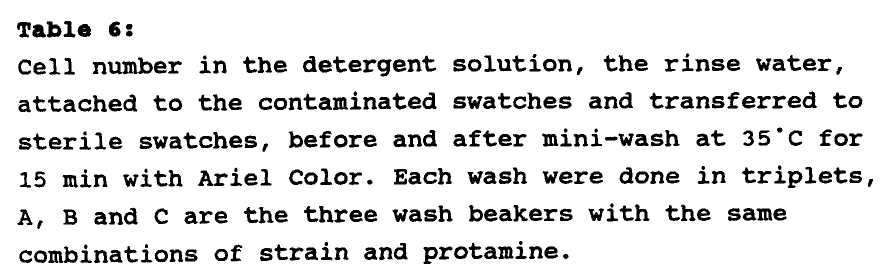

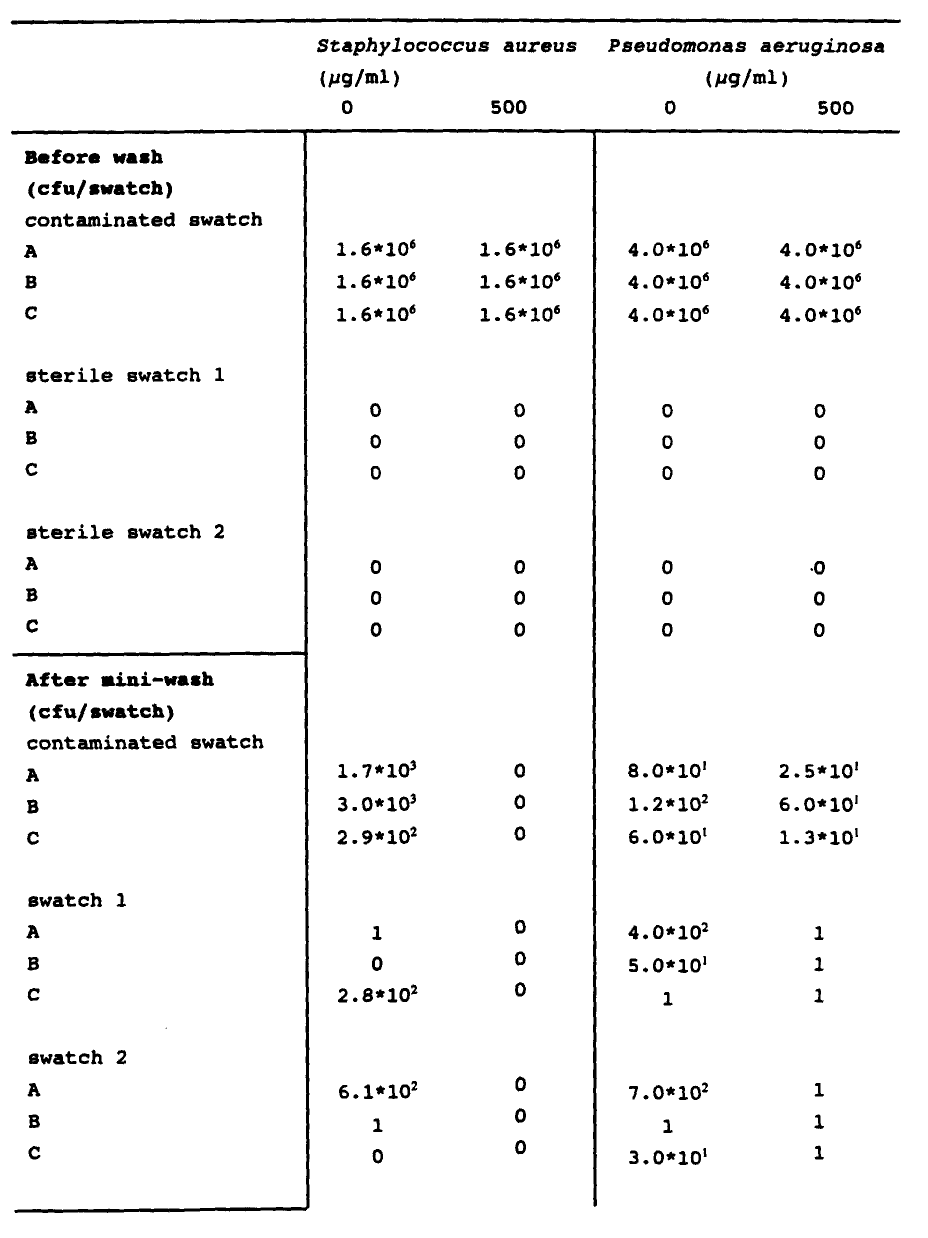

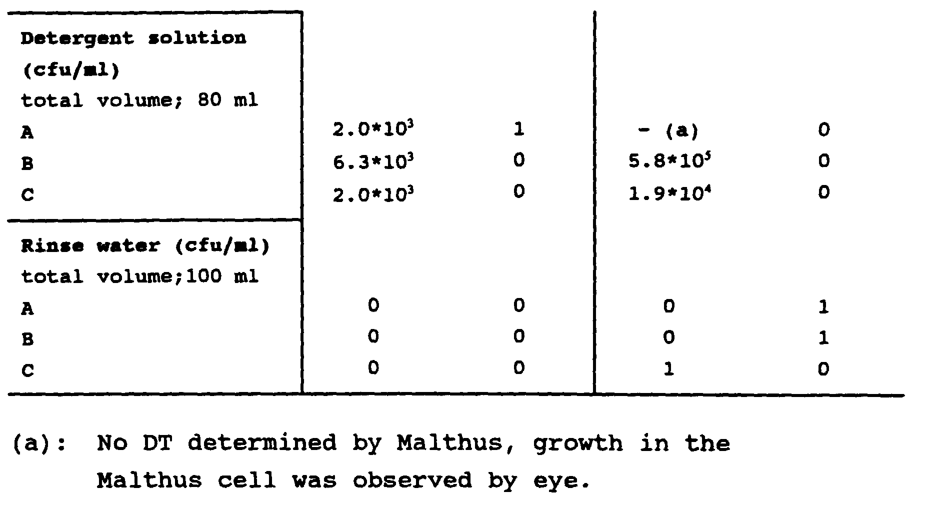

From the results it can be concluded that Ariel Color has no significant bactericidal effect on *S. aureus* and *P*. *aeruginosa* (pathogenic skin isolates).

A high number of cells were washed of the swatches, and found in the detergent solution, and when no protamine were added, cells from the inoculated swatch contaminated the sterile swatches in the wash beaker.

### REFERENCES

Ben Embarek, P. K. & Huss, H. H. 1993 Heat resistance of Listeria monocytogenes in vacuum packaged pasteurized fish fillets. *International Journal of Food Microbiology* **20**, 85-95.
Bierbaum, G. & Sahl, H. G. 1987 Autolytic system of Staphylococcus simulans 22: Influence of cationic peptides on activity of n-acetylmuramoyl-L-alanine amidase. *Journal of Bacteriology* **169**, 5452-5458.
Bierbaum, G. & Sahl, H. G. 1991 Induction of autolysis of Staphylococcus simulans 22 by Pep5 and nisin and influence of the cationic peptides on the activity of the autolytic enzymes. In *Nisin and novel antibiotics* ed. Jung, G. & Sahl, H. G, p. 347-358. ESCOM, Leiden.
Brange, J. 1987 *Galenics of insulin*. Ch. 3, pp. 34-36. Berlin: Springer-Verlag.
Christensen, B., Fink, J., Merrifield, R. B. & Mauzerall, D. 1988 Channel-forming properties of cecropins and related model compounds incorporated into planar lipid membranes. *Proceedings of the National Academy of Sciences of the United States of America* **85**, 5072-5076.
Chung, Steen & Hansen, (1994). Subtilisin gene of Bacillus subtilis ATCC 6633 is incoded in an operon that contains homologues of the hemolysin B transport protein, J. Bact., Vol. 174 (1994), p. 1414-1422.
Connolly, P., Bloomfield, S. F. & Denyer, S. P. 1993 A study of the use of rapid methods for preservative efficacy testing of pharmaceuticals and cosmetics. *Journal of Applied Bacteriology* **75**, 456-462.
Farrag, S. A. & Marth, E. H. 1992 Escherichia coli 0157:H7, Yersinia enterocolitica and their control in milk by the lactoperoxidase system: a review. *Lebensmittel-Wissenschaft und Technologie* **25**, 201-211.
Firstenberg-Eden, R. & Eden, G. 1984 *Impedance microbiology*. Letchworth: John Wiley & Sons Inc.
Gram, L., Wedell-Nedergaard, C. & Huss, H. H. 1990 The bacteriology of fresh and spoiling Lake Victorian Nile perch (Lates niloticus). *International Journal of Food Microbiology* **10**, 303-316.
Hitsch, J. G. 1958 Bactericidal action of histone. *Journal* of experimental medicine **108**, 925-944.
Horrow, J. C. 1985 Protamine: A review of its toxicity. *Anaesthesia Analgesia* **64**, 51-55.
Hugo, W. B. 1978 Membrane-active antimicrobial drugs - a reappraisal of their mode of action in the light of the chemosmotic theory. *International Journal of Pharmaceutics* **1**, 127-131.
Islam, N. MD., Itakura, T. & Motohiro, T. 1984 Antibacterial spectra and minimum inhibition concentration of clupeine and salmine. *Bulletin of the Japanese Society of Scientific Fisheries* **50**, 1705-1708.
Jaques, L. B. 1973 Protamine - antagonist to heparin. *Canadian Medical Association Journal* **108**, 1291-1297.
Jeong, D. K., Harrison, M. A., Frank, J. F. & Wicker, L. 1992 Trials on the antibacterial effect of glucose oxidase on chicken breast skin and muscle. *Journal of Food safety* **13**, 43-49.
Jeppesen, V. F. & Huss, H. H. 1993 Characteristics and antagonistic activity of lactic acid bacteria isolated from chilled fish products. *International Journal of Food Microbiology* **18**, 305-320.
Johansen, C., Gram, L. & Meyer, A. S. 1994 The combined inhibitory effect of lysozyme and low pH on the growth of Listeria monocytogenes. *Journal of Food Protection*, in press.
Jørgensen, B. R. 1986 Sorbic acid - effect on fish - influence of pH on the effect. Masters thesis (in Danish). Technological Laboratory, Danish Ministry of Fisheries, Technological University of Denmark.
Jørgensen, B. R. & Huss, H. H. 1989 Growth and activity of Shewanella putrefaciens isolated from spoiling fish. *International Journal of Food Microbiology* **9**, 51-62.
Kagan, B. L., Selsted, m. E., Ganz, T. & Lehrer, R. I. 1990 Antimicrobial defensin peptides from voltage-dependent ion-permeable channels in planar lipid bilayer membranes. *Proceedings of the National Academy of Sciences of the United States of America* **87**, 210-214.
Knøchel, S. 1989 Aeromonas spp. - ecology and significance in food and water hygiene. Ph.D. thesis. Technological Laboratory and the Royal Veterinary and Agricultural University, Frederiksberg, Denmark.
Kossel, A. 1928 The protamines and histones, Longmans, Green and Co., London, New York, Toronto, 1-63.
Lehrer, R. I., Lichtentstein, A. K. & Ganz, T. 1993 Defensins: Antimicrobial and cytotoxic peptides of animal cells. *Annual Review of Immunology* **11**, 105-128.
Nakae, T. 1985 Outer-membrane permeability of bacteria. *In Critical Reviews in Microbiology* ed. O'Leary, W. M., pp. 1-62. Boca Raton, Florida: CRC Press, Inc.
Phillips, L. G., Yang, S. T., Schulman, W. & Kinsella, J. E. 1989 Effect of lysozyme, clupeine, and sucrose on the foaming properties of whey protein isolate and β-lactaglobulin. *Journal of Food Science* **54**, 743-747.
Rodman, T. C., Pruslin, F. H. & Allfrey, V. G. 1984 Protamine-DNA association in mammalian Spermatozoa. *Experimental Cell Research* **150**, 269-281.
Sahl, H. G. 1987 Influence of the staphylococci-like peptide Pep5 on membrane potential of bacterial cells and cytoplasmic membrane vesicles. *Journal of Bacteriology* **162**, 833-836.
Trevors, J. T. 1986 Bacterial growth and activity as indicators of toxicity. In *Toxicity testing using microorganisms* ed. Bitton, G. & Dutka, B. J. Boca Raton, Florida: CRC Press, Inc.
Vaara, M. 1992 Agents that increase the permeability of the outer membrane. *Microbiological Reviews* **56**, 395-411.
Vaara, M. & Vaara, T. 1983 Polycations as outer membrane-disorganizing agents. *Antimicrobial Agents and Chemotherapy* **24**, 114-122.
Weisner, B. 1984 Lysozyme (muramidase). In *Methods of Enzymatic Analysis* Vol. 4, ed. Bergmeyer, H. U. pp. 189-195.
Weinheim: Verlag Chemie.
Yanagimoto, T., Tanaka, M. & Nagashima, Y. 1992 Changes in the antibacterial activity of salmine during Its Maillard reaction. *Bulletin of the Japanese Society of Scientific Fisheries* **58**, 2153-2158.

## Claims

1. A bactericidal, bacteriostatic, fungicidal and/or fungistatic composition comprising or consisting essentially of a basic protein selected from the group consisting of protamines, protamine sulphates, defensins, magainins, melittin, cecropins and protegrins or a basic peptide selected from the group consisting of polylysins and polyarginins and co-polymers thereof capable of killing microbial cells in combination with an oxidoreductase.

2. The composition according to claim 1 which further comprises a cell-wall degrading enzyme capable of degrading peptidoglucan, chitin and/or teichoic acid, preferably selected from the group consisting of endoglycosidases Type II, lysozymes and chitinases.

3. The composition according to claim 1 or 2, wherein the basic protein has an amino acid sequence consisting of amino acids normally occurring in mammalian cells.

4. The composition according to any of the claims 1-3, wherein the basic protein is of biological or microbiological origin.

5. The composition according to any of the claims 1-4, wherein the basic protein is recovered from biological material.

6. The composition according to any of the claims 1-4, wherein the basic protein is a recombinant protein.

7. The composition according to any of the claims 1-6, wherein the oxidoreductase is selected from the group consisting of oxidases (EC 1.10.3) and peroxidases (EC 1.11.1), preferably from peroxidase enzyme systems (EC 1.11.1.7) and laccase enzymes (EC 1.10.3.2).

8. The composition according to any of the claims 1-7, wherein the peroxidase enzyme system comprises at least one peroxidase enzyme and a hydrogen peroxide generating enzyme system such as an oxidase and a substrate for the oxidase or an amino acid oxidase and a suitable amino acid, or a peroxycarboxylic acid or a salt thereof.

9. A cleaning or detergent composition comprising a basic protein selected from the group consisting of protamines, protamine sulphates, defensins, magainins, melittin, cecropins and protegrins or a basic peptide selected from the group consisting of polylysins and polyarginins and co-polymers thereof capable of killing microbial cells, an oxidoreductase, a surfactant and optionally a microbial cell-wall degrading enzyme capable of degrading peptidoglucan, chitin and/or teichoic acid.

10. The composition according to claim 9, wherein the basic protein has an amino acid sequence consisting of amino acids normally occurring in mammalian cells.

11. The composition according to any of the claims 9-10, wherein the cell-wall degrading enzyme is selected from the group consisting of endoglycosidases Type II and muramidases such as lysozymes and chitinases; and/or the oxidoreductase is selected from peroxidase enzyme systems (EC 1.11.1.7) and laccase enzymes (EC 1.10.3.2).

12. The composition according to any of the claims 9-11, wherein the peroxidase system comprises at least one peroxidase enzyme and a hydrogen peroxide generating enzyme system such as an oxidase and a substrate for the oxidase or an amino acid oxidase and a suitable amino acid, or a peroxycarboxylic acid or a salt thereof.

13. The composition according to any of the claims 9-12, which further comprises at least one enzyme selected from the group consisting of proteases, amylases, cellulases, and lipases.

14. The composition according to any of the claims 9-13, wherein the surfactant is a detergent surfactant, preferably selected from the group consisting of anionic, nonionic, ampholytic, zwitterionic and cationic surfactants.

15. The composition according to any of the claims 9-14, wherein the basic protein is a protamine or a protamine sulphate in an amount effective for killing cells or inhibiting growth of cells, preferably in an amount corresponding to between 1 and 4000 mg per 1 cleaning liquor or washing liquor, more preferably between 1 and 2000 mg per 1 cleaning liquor or washing liquor, especially between 5 and 1000 mg per 1 cleaning liquor or washing liquor.

16. A method for killing microbial cells present on a hard surface comprising contacting the surface with a cleaning composition according to any of the claims 9-15 or a composition according to any of the claims 1-8, preferably a composition comprising a protamine or a protamine sulphate.

17. A method for killing microbial cells or inhibiting growing microbial cells present on laundry comprising contacting the laundry with a detergent composition according to any of the claims 9-15 or a composition according to any of the claims 1-8, preferably a composition comprising a protamine or a protamine sulphate.

18. A method for preservation of food, beverages, cosmetics, contact lens products, food ingredients or enzyme compositions comprising incorporating into the unpreserved food, beverages, cosmetics, contact lens products, food ingredients or enzyme compositions a basic protein selected from the group consisting of protamines, protamine sulphates, defensins, magainins, melittin, cecropins and protegrins or a basic peptide selected from the group consisting of polylysins and polyarginins and co-polymers thereof or a composition according to any of the claims 1-8 in an amount effective for inhibiting growing microbial cells, preferably a protamine or a protamine sulphate or a composition comprising a protamine or a protamine sulphate.

19. Use of a composition according to any of the claims 1-8 or a cleaning composition according to any of the claims 9-15 for preparing a disinfectant useful for killing microbial cells present on human or animal skin, mucous membranes, wounds, bruises or in the eye and comprising a basic protein selected from the group consisting of protamines, protamine sulphates, defensins, magainins, melittin, cecropins and protegrins or a basic peptide selected from the group consisting of polylysins and polyarginins and co-polymers thereof in an amount effective for killing the cells, preferably a protamine or protamine sulphate, or a composition according to any of the claims 1-8 or 9-15.

## Patentansprüche

1. Bakterizide, bakteriostatische, fungizide und/oder fungistatische Zusammensetzung, welche ein basisches Protein, das aus der Gruppe, bestehend aus Protaminen, Protaminsulfaten, Defensinen, Magaininen, Melittin, Cecropinen und Protegrinen, ausgewählt ist, oder ein basisches Peptid, das aus der Gruppe, bestehend aus Polylysinen und Polyargininen und Copolymeren davon, ausgewählt ist, welches Protein oder Peptid zur Abtötung von mikrobiellen Zellen imstande ist, in Kombination mit einer Oxidoreduktase umfaßt oder im wesentlichen daraus besteht.

2. Zusammensetzung nach Anspruch 1, welche ferner ein zellwandabbauendes Enzym, das zum Abbau von Peptidoglucan, Chitin und/oder Teichonsäure in der Lage ist, vorzugsweise aus der Gruppe, bestehend aus Endoglycosidasen vom Typ II, Lysozymen und Chitinasen, ausgewählt, umfaßt.

3. Zusammensetzung nach Anspruch 1 oder 2, worin das basische Protein eine Aminosäuresequenz aufweist, die aus normalerweise in Säugerzellen vorkommenden Aminosäuren besteht.

4. Zusammensetzung nach irgendeinem der Ansprüche 1-3, worin das basische Protein biologischen oder mikrobiologischen Ursprungs ist.

5. Zusammensetzung nach irgendeinem der Ansprüche 1-4, worin das basische Protein aus biologischem Material gewonnen ist.

6. Zusammensetzung nach irgendeinem der Ansprüche 1-4, worin das basische Protein ein rekombinantes Protein ist.

7. Zusammensetzung nach irgendeinem der Ansprüche 1-6, worin die Oxidoreduktase aus der Gruppe, bestehend aus Oxidasen (EC 1.10.3) und Peroxidasen (EC 1.11.1), vorzugsweise aus Peroxidase-Enzymsystemen (EC 1.11.1.7) und Laccase-Enzymen (EC 1.10.3.2) ausgewählt ist.

8. Zusammensetzung nach irgendeinem der Ansprüche 1-7, worin das Peroxidase-Enzymsystem mindestens ein Peroxidase-Enzym und ein Wasserstoffperoxid erzeugendes Enzymsystem, wie z.B. eine Oxidase und ein Substrat für die Oxidase oder eine Aminosäureoxidase und eine geeignete Aminosäure, oder eine Peroxycarbonsäure oder ein Salz davon umfaßt.

9. Reinigungs- oder Detergenszusammensetzung, welche ein basisches Protein, das aus der Gruppe, bestehend aus Protaminen, Protaminsulfaten, Defensinen, Magaininen, Melittin, Cecropinen und Protegrinen, ausgewählt ist, oder ein basisches Peptid, das aus der Gruppe, bestehend aus Polylysinen und Polyargininen und Copolymeren davon, ausgewählt ist, welches Protein oder Peptid zur Abtötung von mikrobiellen Zellen imstande ist, eine Oxidoreduktase, ein Tensid und gegebenenfalls ein mikrobielle Zellwände abbauendes Enzym, das zum Abbau von Peptidoglucan, Chitin und/oder Teichonsäure in der Lage ist, umfaßt.

10. Zusammensetzung nach Anspruch 9, worin das basische Protein eine Aminosäuresequenz aufweist, die aus normalerweise in Säugerzellen vorkommenden Aminosäuren besteht.

11. Zusammensetzung nach irgendeinem der Ansprüche 9-10, worin das zellwandabbauende Enzym aus der Gruppe, bestehend aus Endoglycosidasen vom Typ II und Muramidasen, wie z.B. Lysozyme und Chitinasen, ausgewählt ist; und/oder die Oxidoreduktase aus Peroxidase-Enzymsystemen (EC 1.11.1.7) und Laccase-Enzymen (EC 1.10.3.2) ausgewählt ist.

12. Zusammensetzung nach irgendeinem der Ansprüche 9-11, worin das Peroxidase-System mindestens ein Peroxidase-Enzym und ein Wasserstoffperoxid erzeugendes Enzymsystem, wie z.B. eine Oxidase und ein Substrat für die Oxidase oder eine Aminosäureoxidase und eine geeignete Aminosäure, oder eine Peroxycarbonsäure oder ein Salz davon umfaßt.

13. Zusammensetzung nach irgendeinem der Ansprüche 9-12, welche ferner mindestens ein Enzym umfaßt, das aus der Gruppe, bestehend aus Proteasen, Amylasen, Cellulasen und Lipasen, ausgewählt ist.

14. Zusammensetzung nach irgendeinem der Ansprüche 9-13, worin das Tensid ein Detergens-Tensid ist, das vorzugsweise aus der Gruppe, bestehend aus anionischen, nichtionischen, ampholytischen, zwitterionischen und kationischen Tensiden, ausgewählt ist.

15. Zusammensetzung nach irgendeinem der Ansprüche 9-14, worin das basische Protein ein Protamin oder Protaminsulfat in einer zur Abtötung von Zellen oder zur Hemmung des Wachstums von Zellen wirksamen Menge ist, vorzugsweise in einer Menge, die 1 bis 4000 mg/l Reinigungslauge oder Waschlauge, bevorzugter 1 bis 2000 mg/l Reinigungslauge oder Waschlauge, insbesondere 5 bis 1000 mg/l Reinigungslauge oder Waschlauge, entspricht.

16. Verfahren zur Abtötung von mikrobiellen Zellen, die auf einer harten Oberfläche vorliegen, umfassend das Kontaktieren der Oberfläche mit einer Reinigungszusammensetzung nach irgendeinem der Ansprüche 9-15 oder einer Zusammensetzung nach irgendeinem der Ansprüche 1-8, vorzugsweise einer Zusammensetzung, die ein Protamin oder ein Protaminsulfat umfaßt.

17. Verfahren zur Abtötung mikrobieller Zellen oder zur Hemmung wachsender mikrobieller Zellen, die auf Wäsche vorliegen, umfassend das Kontaktieren der Wäsche mit einer Detergenszusammensetzung nach irgendeinem der Ansprüche 9-15 oder einer Zusammensetzung nach irgendeinem der Ansprüche 1-8, vorzugsweise einer Zusammensetzung, die ein Protamin oder ein Protaminsulfat umfaßt.

18. Verfahren zur Konservierung von Nahrungsmitteln, Getränken, Kosmetika, Kontaktlinsen-Produkten, Nahrungsmittelkomponenten oder Enzymzusammensetzungen, umfassend die Inkorporation eines basischen Proteins, das aus der Gruppe, bestehend aus Protaminen, Protaminsulfaten, Defensinen, Magaininen, Melittin, Cecropinen und Protegrinen, ausgewählt ist, oder eines basischen Proteins, das aus der Gruppe, bestehend aus Polylysinen und Polyargininen und Copolymeren davon, ausgewählt ist, oder einer Zusammensetzung nach irgendeinem der Ansprüche 1-8 in einer zur Hemmung wachsender mikrobieller Zellen wirksamen Menge, vorzugsweise eines Protamins oder eines Protaminsulfats oder einer Zusammensetzung, die ein Protamin oder ein Protaminsulfat umfaßt, in die nicht-konservierten Nahrungsmittel, Getränke, Kosmetika, Kontaktlinsen-Produkte, Nahrungsmittelkomponenten oder Enzymzusammensetzungen.

19. Verwendung einer Zusammensetzung nach irgendeinem der Ansprüche 1-8 oder einer Reinigungszusammensetzung nach irgendeinem der Ansprüche 9-15 zur Herstellung eines Desinfektionsmittels, das sich zur Abtötung mikrobieller Zellen, die auf menschlicher oder tierischer Haut, Schleimhäuten, Wunden, Quetschungen oder im Auge vorliegen, eignet und ein basisches Protein, das aus der Gruppe, bestehend aus Protaminen, Protaminsulfaten, Defensinen, Magaininen, Melittin, Cecropinen und Protegrinen ausgewählt ist, oder ein basisches Peptid, das aus der Gruppe, bestehend aus Polylysinen und Polyargininen und Copolymeren davon, ausgewählt ist, in einer zur Abtötung der Zellen wirksamen Menge, vorzugsweise ein Protamin oder Protaminsulfat oder eine Zusammensetzung nach irgendeinem der Ansprüche 1-8 oder 9-15, umfaßt.

## Revendications

1. Composition bactéricide, bactériostatique, fongicide et/ou fongistatique comprenant ou se composant essentiellement d'une protéine basique choisie dans le groupe constitué des protamines, sulfates de protamine, défensines, magainines, mélittine, cécropines et protégrines ou d'un peptide basique choisi dans le groupe constitué des polylysines et polyarginines et leurs copolymères capable de tuer des cellules microbiennes en combinaison avec une oxydoréductase.

2. Composition selon la revendication 1 qui comprend en outre une enzyme de dégradation des parois cellulaires capable de dégrader le peptidoglycane, la chitine et/ou l'acide téichoïque, choisie de préférence dans le groupe constitué des endoglycosidases de type II, des lysozymes et des chitinases.

3. Composition selon la revendication 1 ou 2, dans laquelle la protéine basique a une séquence d'acides aminés constituée d'acides aminés que l'on trouve normalement dans les cellules de mammifères.

4. Composition selon l'une quelconque des revendications 1 à 3, dans laquelle la protéine basique est d'origine biologique ou microbiologique.

5. Composition selon l'une quelconque des revendications 1 à 4, dans laquelle la protéine basique est récupérée à partir de matière biologique.

6. Composition selon l'une quelconque des revendications 1 à 4, dans laquelle la protéine basique est une protéine recombinante.

7. Composition selon l'une quelconque des revendications 1 à 6, dans laquelle l'oxydoréductase est choisie dans le groupe constitué des oxydases (EC 1.10.3) et des peroxydases (EC 1.11.1), de préférence parmi les systèmes enzymatiques de peroxydase (EC 1.11.1.7) et les enzymes laccases (EC 1.10.3.2).

8. Composition selon l'une quelconque des revendications 1 à 7, dans laquelle le système enzymatique de peroxydase comprend au moins une enzyme peroxydase et un système enzymatique générant du peroxyde d'hydrogène, tel qu'une oxydase et un substrat pour l'oxydase ou une aminoacide oxydase et un acide aminé approprié, ou un acide peroxycarboxylique ou un de ses sels.

9. Composition nettoyante ou détergente comprenant une protéine basique choisie dans le groupe constitué des protamines, sulfates de protamine, défensines, magainines, mélittine, cécropines et protégrines ou un peptide basique choisi dans le groupe constitué des polylysines et polyarginines et leurs copolymères capable de tuer des cellules microbiennes, une oxydoréductase, un tensioactif et éventuellement une enzyme de dégradation des parois cellulaires microbiennes capable de dégrader le peptidoglycane, la chitine et/ou l'acide téichoïque.

10. Composition selon la revendication 9, dans laquelle la protéine basique a une séquence d'acides aminés constituée d'acides aminés que l'on trouve normalement dans les cellules de mammifères.

11. Composition selon l'une quelconque des revendications 9 et 10, dans laquelle l'enzyme de dégradation des parois cellulaires est choisie dans le groupe constitué des endoglycosidases de type II et des muramidases telles que les lysozymes et les chitinases ; et/ou l'oxydoréductase est choisie parmi les systèmes enzymatiques de peroxydase (EC 1.11.1.7) et les enzymes laccases (EC 1.10.3.2).

12. Composition selon l'une quelconque des revendications 9 à 11, dans laquelle le système de peroxydase comprend au moins une enzyme peroxydase et un système enzymatique générant du peroxyde d'hydrogène, tel qu'une oxydase et un substrat pour l'oxydase ou une aminoacide oxydase et un acide aminé approprié, ou un acide peroxycarboxylique ou un de ses sels.

13. Composition selon l'une quelconque des revendications 9 à 12, qui comprend en outre au moins une enzyme choisie dans le groupe constitué des protéases, des amylases, des cellulases et de lipases.

14. Composition selon l'une quelconque des revendications 9 à 13, dans laquelle le tensioactif est un tensioactif détergent, de préférence choisi dans le groupe constitué des tensioactifs anioniques, non ioniques, ampholytiques, zwittérioniques et cationiques.

15. Composition selon l'une quelconque des revendications 9 à 14, dans laquelle la protéine basique est une protamine ou un sulfate de protamine en une quantité efficace pour tuer des cellules ou inhiber la croissance de cellules, de préférence en une quantité correspondant à entre 1 et 4 000 mg par litre de liqueur de nettoyage ou de liqueur de lavage, plus préférentiellement entre 1 et 2 000 mg par litre de liqueur de nettoyage ou de liqueur de lavage, en particulier entre 5 et 1 000 mg par litre de liqueur de nettoyage ou de liqueur de lavage.

16. Procédé pour tuer des cellules microbiennes présentes sur une surface dure, comprenant la mise en contact de la surface avec une composition nettoyante selon l'une quelconque des revendications 9 à 15, ou une composition selon l'une quelconque des revendications 1 à 8, de préférence une composition comprenant une protamine ou un sulfate de protamine.

17. Procédé pour tuer des cellules microbiennes ou inhiber des cellules microbiennes en croissance présentes sur du linge, comprenant la mise en contact du linge avec une composition détergente selon l'une quelconque des revendications 9 à 15 ou une composition selon l'une quelconque des revendications 1 à 8, de préférence une composition comprenant une protamine ou un sulfate de protamine.

18. Procédé pour la conservation d'aliments, de boissons, de cosmétiques, de produits de type lentille de contact, d'ingrédients alimentaires ou de compositions enzymatiques, comprenant l'incorporation dans les aliments, boissons, cosmétiques, produits de type lentille de contact, ingrédients alimentaires ou compositions enzymatiques, non conservé(e)s, d'une protéine basique choisie dans le groupe constitué des protamines, sulfates de protamine, défensines, magainines, mélittine, cécropines et protégrines ou d'un peptide basique choisi dans le groupe constitué des polylysines et polyarginines et leurs copolymères ou d'une composition selon l'une quelconque des revendications 1 à 8 en une quantité efficace pour inhiber des cellules microbiennes en croissance, de préférence une protamine ou un sulfate de protamine ou une composition comprenant une protamine ou un sulfate de protamine.

19. Utilisation d'une composition selon l'une quelconque des revendications 1 à 8 ou d'une composition nettoyante selon l'une quelconque des revendications 9 à 15 pour préparer un désinfectant utile pour tuer des cellules microbiennes présentes sur la peau, les membranes muqueuses, les plaies, les contusions, humaines ou animales ou dans l'oeil humain ou animal, et comprenant une protéine basique choisie dans le groupe constitué des protamines, sulfates de protamine, défensines, magainines, mélittine, cécropines et protégrines ou un peptide basique choisi dans le groupe constitué des polylysines et polyarginines et leurs copolymères en une quantité efficace pour tuer les cellules, de préférence une protamine ou un sulfate de protamine, ou une composition selon l'une quelconque des revendications 1 à 8 ou 9 à 15.
